# EUROPEAN PATENT SPECIFICATION

(11) **EP 1 827 577 B1**
(45) Date of publication and mention of the grant of the patent: **24.09.2008**
(21) Application number: 05813244.0
(22) Date of filing: 13.10.2005
(51) Int. Cl.: A61N 1/05, A61N 1/30

(54) **SELF-FIXATING IMPLANTABLE SCAFFOLDS FOR THE ADMINISTRATION OF BIOLOGICAL OR PHARMACEUTICAL SUBSTANCES**
SELBSTFIXIERENDE IMPLANTIERBARE GERÜSTE ZUR VERABREICHUNG VON BIOLOGISCHEN ODER PHARMAZEUTISCHEN SUBSTANZEN
SQUELETTES AUTOFIXANTS IMPLANTABLES POUR L'ADMINISTRATION DES AGENTS BIOLOGIQUES OU PHARMACEUTIQUES

(30) Priority: 13.10.2004 US 618425 P
(43) Date of publication of application: 05.09.2007
(73) Proprietor: MEDTRONIC, INC., Minneapolis, MN 55432 (US)
(72) Inventor: SIGG, Daniel, C., St. Paul, Minnesota 55108 (US); HINIDUMA-LOKUGE, Prasanga , D., Minneapolis, Minnesota 55403 (US); SHARMA, Vinrod, Blaine, Minnesota 55449 (US); SOMMER, John, L., Coon Rapids, Minnesota 55449 (US)
(74) Representative: Hughes, Andrea Michelle
(86) International application number: PCT/US2005/037430
(87) International publication number: WO 2006/044890

(56) References cited:
- EP-A- 0 422 363
- WO-A-01/10348
- WO-A-98/15317
- WO-A-03/003948
- US-A1- 2001 014 813
- US-A1- 2003 073 972
- US-A1- 2003 233 101
- US-A1- 2004 098 030

## Description

The present invention relates to scaffolds for implantation into body tissues. In particular, the present invention relates to self-fixating scaffolds that hold therapeutic material for implantation into body tissues.

Direct implantation of therapeutic material into tissues to treat various diseases and conditions offers immediate and/or long-term solutions. The therapeutic material is typically injected into the target tissue with needles and catheters. Injection, though a reasonable route of delivery, is not without challenges. In the heart, about 80% to 90% of injectate is lost via venous and lymphatic pathways and mechanical means. The delivery process, therefore, is inefficient. The ultimate destination of the leaked therapeutic material is unknown and may result in ineffective treatment or deleterious effects. In addition, therapeutic material that is retained in the tissue may be eventually flushed out due to lack of an adequate substrate for binding.

If cells are injected, about 80% to 90% of the delivered cells undergo cell death due to lack of vasculature and necessary nutrients and gaseous exchange within the injected tissue. Scaffolds have been used to deliver biological materials to target tissues to overcome the shortcomings of injections. Scaffolds, however, if not sutured in place, can become dislodged. Thus, there isa need for a better method of delivering therapeutic material to target tissues.

The present invention is a self-fixating scaffold as defined in claim 1. The self fixating scaffold includes a scaffold to hold therapeutic material.

Self-fixating scaffold devices according to the preamble of claim 1 are known from e.g. US2003/0233101 A1.
Figure 1 is a schematic illustration of a first embodiment of a self-fixating scaffold given by way of background.
Figure 2 is a schematic illustration of a second embodiment of a self-fixating scaffold given by way of background.
Figure 3 is a schematic illustration of a third embodiment of a self-fixating scaffold given by way of background.
Figure 4 is a schematic illustration of a self-fixating scaffold according to the present invention.
Figure 5 is a schematic illustration of a self-fixating scaffold given by way of background.
Figure 6a is a schematic side view of a self-fixating scaffold given by way of background.
Figure 6b is a schematic bottom view of a self-fixating scaffold given by way of background.

The present invention is a self-fixating scaffold that is useful in the delivery of therapeutic material to various tissues of the body. The self-fixating scaffold essentially includes a scaffold to hold therapeutic material.

The invention is applicable for a variety of treatments such as cell therapy, gene therapy, protein therapy, and drug therapy. The therapeutic material, it follows, may be cells, viral vectors, nucleic acid, peptide/protein, pharmaceutical drugs, or any combination of these.

Examples of diseases/conditions that may be treated with a therapeutic material delivered by a self-fixating scaffold include the following: bradyarrhythmias-deliver a biological pacemaker, reconstruct the AV node; tachyarrhythmias--deliver biological material to enhance electrical conduction across the border zone of an infarct or induce conduction block (bioablation using fibroblasts or other cells); other arrhythmiasshort-term drug delivery after cardiac surgery to avoid post-op atrial fibrillation (attachable epicardially); heart failure-deliver growth factors to induce cardiomyocyte growth and proliferation; other cardiac diseases such as coronary artery disease--deliver biological material for angiogenesis and arteriogenesis; other diseases such as focal neurological diseases--deliver miniaturized scaffolds for stem cell therapy (dopamine producing stem cells for Parkinson, scaffolds releasing NGF for stroke, seeding neural stem cells to treat stroke, see K.I. Park et al., Nat Biotechnol 20(11):1111-7); and diabetes--deliver insulin-producing stem cells that could be attached anywhere in the body. Other uses include tissue regeneration for diseased or damaged tissue including bone and cancer treatment.

Any of a number of techniques known in the art may be used to fabricate self-fixating scaffolds of the present invention. Examples include the following: fiber bonding, solvent-casting and particulate leaching, membrane lamination, melt molding, emulsion/freeze-drying, high pressure (gas foaming), and phase inversion. Various methods are also described in R.C. Thomson, et al., Biodegradable Polymer Scaffolds to Regenerate Organs, Adv. Polym. Sci. 122, 245, (1995) and C. E. Holy et al., Bone Tissue Engineering on Biodegradable Polymers: Preparation of a Novel Poly(lactide-co-glycolide) Foam, in Biomaterials, Carriers for Drug Delivery and Scaffolds for Tissue Engineering. New York: AIChE; 1997. p. 272-4.

Various constraints of the scaffolds should be considered in the fabrication process. The scaffolds have a pore size that provides a large enough surface area for cells to adhere, while encouraging ingrowth of blood vessels. The scaffolds allow for effective nutrient exchange (mass transfer). Typically, the diameter of the pore is between about 30 µm and 120 µm. Overall pore size determines physical and electrical connectivity, which is important in some therapies.

Porosity (number of pores as opposed to pore size) enhances gene delivery and is controlled by various means. For example, when scaffold material is dissolved in solvents followed by solvent evaporation, the rate of solvent evaporation is varied to form the desired pore size and porosity of the scaffold. Varying solvent volume relative to evaporation rate can also control porosity.

Porosity is also controllable by fabricating the scaffolds in a pressure quench process using carbon dioxide as a blowing agent. The body of the polymer is saturated with carbon dioxide and subsequently removed through vacuum suction under a specified pressure. Varying temperature and pressure of the reaction controls the volume of carbon dioxide absorption, which leads to control of pore quantity and size. See L. Singh et al., Biomaterials 25:2611-17.

The scaffolds are strong enough to resist collapsing under the stress of proliferating cells. Prior art biodegradable scaffolds have collapsed under this type of stress.

Biodegradable scaffolds of the present invention have a degradation rate equal to the rate of cell proliferation of the tissue. The fixation element can be made to either degrade much slower than the rest of the scaffold or be made non-degradable.

Degradation rate is controllable through a number of methods. Manipulation of polymer-chain lengths will vary the degradation rate. The addition of modifying side chains to add hydrophilic/hydrophobic characteristics (i.e. chemical composition) controls the rate of degradation.

Degradation rate is also controlled by making di/tri/penta coblock polymers. This combines properties of two monomers to give a mechanically altered resultant polymer--similar to a metallic alloy.

In one example, Polycaprolactone (PCL) is combined with Polyurethane (PU), which results in a polymer with a higher modulus and greater extensibility than either polymer alone. Polyethylene Oxide (PEO) is combined with PU, which results in a tacky, viscous material. This combination may be useful for fabricating a fixation element.

A second example combines Polyglycolic acid (PGA) and Poly(L-lactic acid) (PLLA). PGA is highly crystalline and hydrophilic, while PLLA is less crystalline and more hydrophobic due to the presence of a methyl group. PLLA degrades at a slower rate than PGA and has good strength properties. Therefore, by controlling the ratio of PLLA:PGA, a Poly(D, L-lactic-co-glycolide) (PLGA) copolymer can be designed to have the desired degradation rate and mechanical strength.

Lastly, the scaffolds and fixation elements should be stiff enough to penetrate tissue. The stiffness required for penetration will vary depending on the tissue in which the self-fixating scaffold will be anchored.

The fixation element can be fabricated of biodegradable or non-biodegradable polymer or metal or any other suitable biocompatible material. PLLA, for example, could be used to fabricate fixation elements having the mechanical properties mentioned above. Nitinol or platinum may be used or other metallic materials. A fixation element described in U.S. Patent No. 5,246,014, assigned to Medtronic, Inc. may also be utilized.

Other considerations for scaffold fabrication may include the following: crystallinity, molecular orientation, surface/volume ratio, molecular weight, molecular weight distribution, and texture. In all cases, however, the scaffold is biocompatible.

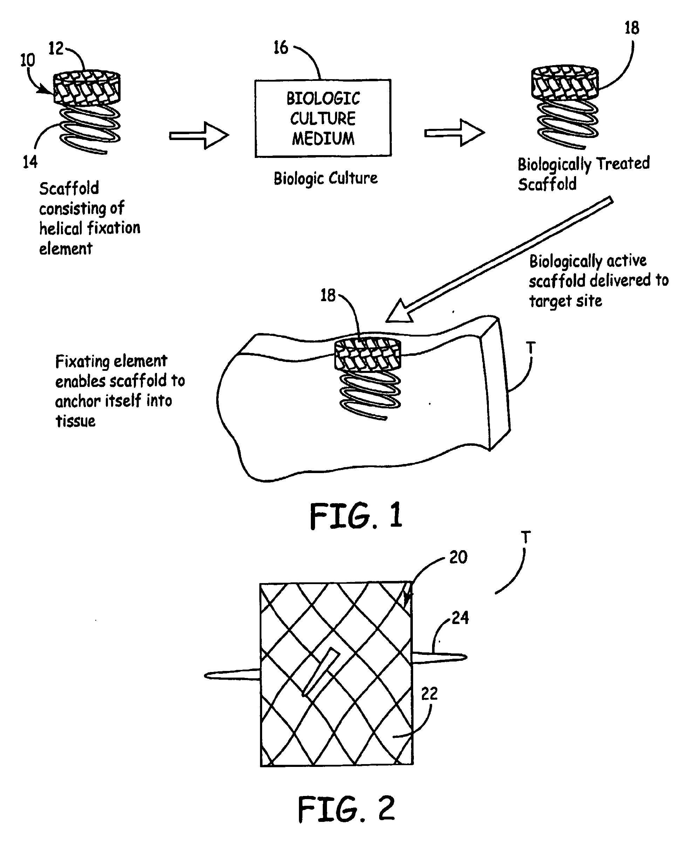

Figure 1 is a first example of a self-fixating scaffold. Figure 1 includes self-fixating scaffold 10 having scaffold 12 and fixation element 14. Scaffold 12 retains or holds the therapeutic material while fixation element 14 anchors self-fixating scaffold 10 into tissue T.

Self-fixating scaffold 10 is fabricated and impregnated with biological material *in vitro.* However, biological material may be delivered to self-fixating scaffold 10 after its delivery to tissue T. Self-fixating scaffold 10 is cultured in culture medium 16. Culture medium 16 contains the biological material, which may also include other factors, such as growth factors and nutrients, to enhance cell viability or chemotactic factors. Once impregnated with the biological material, cultured scaffold 18 is delivered to tissue T. Tissue T may be any body tissue and will vary depending on the therapy being carried out. The self-fixating scaffold is especially useful for implantation into tissues where therapeutic materials are likely to be washed away, such as in and around the heart.

Successful treatment of some cellular therapies may depend in part on incorporation of the implanted cells between cells that form tissue T. Therefore, in one embodiment, chemotactic factors such as insulin-like growth factor-1 (IGF-1) are injected into tissue T in order to pull the cells into tissue T. Fixation element 14 may be hollow such that scaffold 10 is also an injection device.

Figure 2 shows a second example of a self-fixating scaffold. Self-fixating scaffold 20 includes scaffold 22 and arms 24. Arms 24 are spring-loaded or made of a shape-memory material such as nitinol. Upon delivery, arms 24 open and anchor self-fixating scaffold 20 in tissue T.

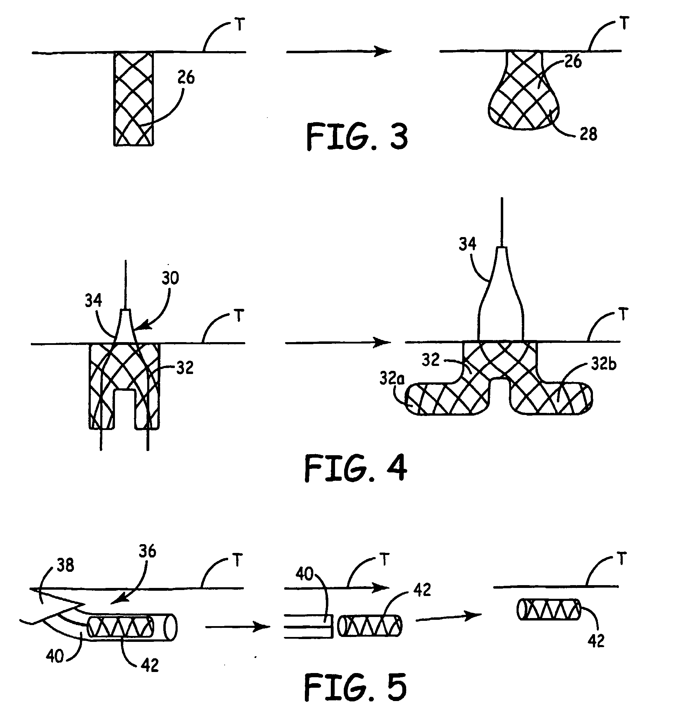

Figure 3 shows a third example of a self-fixating scaffold. Here, scaffold 26 is implanted into tissue T. Scaffold 26 is shaped for ease of implantation as shown in Figure 3. Once implanted, portion 28 of scaffold 26 expands to anchor scaffold 26 into tissue T.

Figure 4 is an embodiment of a self-fixating scaffold according to the present invention. Figure 4 shows scaffold device 30 with scaffold 32 and skeleton 34. Skeleton 34 is formed from a metallic or similarly stiff material. In operation, scaffold device 30 is implanted into tissue T. Skeleton 34 provides stiffness to scaffold 32 for penetrating tissue T. Alternatively, skeleton 34 may also act as a lead for remote control during implantation. Once implanted, skeleton 34 is removed resulting in portions 32a and 32b of scaffold 32 changing form. Portions 32a and 32b anchor scaffold 32 within tissue T after implantation.

Figure 5 shows a further example of a self-fixating scaffold. Scaffold system 36 includes needle 38, catheter 40, and scaffold 42. In operation, scaffold system 36 is inserted into tissue T such that scaffold 42 is nearly parallel to the surface of tissue T. Needle 38 is retracted followed by retraction of catheter 40. Scaffold 42 remains implanted within tissue T.

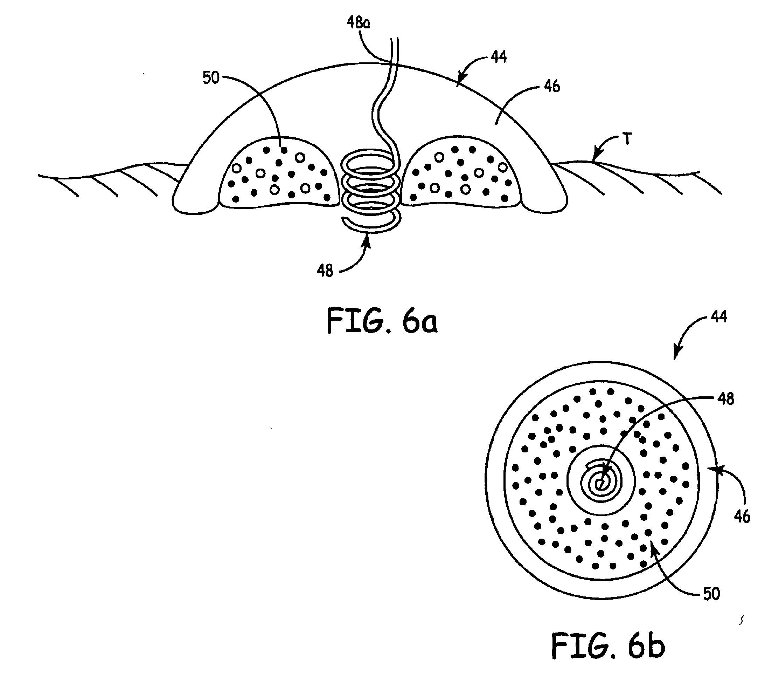

Figures 6a and 6b show another example of a self-fixating scaffold. Self-fixating scaffold 44 includes scaffold 46, fixation element 48 with extension 48a, and therapeutic material 50, which is impregnated in scaffold 46 in a donut distribution pattern. Here, fixation element 48 is metallic and includes extension 48a, which acts as a lead. Extension 48a provides remote control guidance during implantation of self-fixating scaffold 44. A fixation element that may be utilized in this embodiment is described in U.S. Patent No. 5,246,014, assigned to Medtronic, Inc.

The scaffolds shown above are all fabricated *in vitro* and may or may not be impregnated with therapeutic material prior to delivery into tissue T.

If therapeutic material is delivered by injection, spherical scaffolds impregnated with therapeutic material are injected into the tissue. The size of the scaffolds relative to the therapeutic material reduces the chance of the scaffolds being washed away and obstructs their own washout pathways. The spherical scaffold will also initially buffer biological material, such as cells, from the environment of the tissue.

In other cases, scaffolds may be fabricated *in vivo.* An injectable polymer is injected into the tissue. The injectable polymer forms a polymeric scaffold that may be mixed, or impregnated, with the therapeutic material prior to injection.

Upon injection, the injectable polymer undergoes a phase transition that may be driven by temperature or the presence of calcium ions, for example. Thus, once formed, the scaffold is self-fixated within the tissue.

Scaffolds provide several advantages. Scaffolds effectively deliver a predetermined quantity of therapeutic material to tissues and enable focal, localized delivery. Cells impregnated in a scaffold have a matrix within which to grow and will face less stress upon being delivered to tissue, because the scaffold provides a shelter from drastic environmental changes. Nucleic acid may be coaxed to adhere to surfaces of scaffolds. Lastly, the scaffolds act as biomechanical skeletons to reduce leakage of therapeutic material. Self-fixating scaffolds, in particular, may simplify delivery procedures, because no sutures are required to anchor the scaffold.

Scaffolds are also useful for specialized tissues. Cardiac tissue, for example, is an anisotropic tissue. It shows anisotropic propagation of electrical wave fronts--the action potential propagates faster along the direction of the myocardial fibers than transverse to it. The axis of anisotropy rotates along the thickness of the myocardium from the endocardial to the epicardial layer. The scaffolds are engineered with a specific lattice structure for directed cell growth such that cells implanted in cardiac tissue have desired anisotropy. The architecture of scaffold fibers are specifically arranged to encourage cellular anisotropy. The prior art includes methods of using self-assembling oligopeptide monolayers to align cells in specific manners and patterns. See, for example, S. Zhang et al., Biomaterials 20:1213-20. Cells injected without a scaffold presumably integrate randomly into the tissue, thus forming an isotropic structure. Scaffolds aid in overcoming random integration to increase the success of electrically repairing cardiac tissue.

Scaffolds of the present invention may be fabricated from any one or more of a number of biodegradable and non-biodegradable polymers. Because the fixation elements of the scaffolds need to penetrate the tissue and be robust enough to resist being dislodged from the tissue, these are fabricated from a different polymer than that of the scaffold portion that holds the therapeutic material. In some instances, the fixation element is fabricated from a metallic material such as platinum. Examples of polymers that may be used in fabricating the self-fixating scaffolds include the following: synthetic polymers such as PLA:poly(lactic acid), PGA:poly(glycolic acid), PLGA:poly(D, L-lactic-co-glycolide), PEG:polyethylene glycol, PCL:poly(e-caprolactone), PLLA, polyurethane-PCL, polyurethane- PEO:poly ethylene oxide, PCLA:polymer of e-caprolactone-co-L-lactide reinforced with knitted poly-L-lactide fabric, and diblock, triblock, and pentablock copolymer variants of the above; natural polymers such as alginate, collagen, starch/cellulose, cellulose acetate, fibrin, platelet gel, hydrogel, gelatin, chitin, pectin, and hyaluronic acid polymers; combination products such as collagen/PLA, pectin/PLGA, and chitin/PLGA; and injectable polymers such as oligopeptides, alginates, fibrin, and platelet gel (thrombin and platelet combination).

Scaffolds fabricated from synthetic-natural copolymer variants have useful properties that incorporate properties of all-synthetic and all-natural scaffolds. Synthetic polymers lack cell-recognition signals. Naturally-derived polymers rapidly remodel in the body, at times undergoing rapid hydrolysis. Therefore, a mixture of synthetic and natural polymers creates scaffolds that possess the advantageous features of both.

Implantation of self-fixating scaffolds can utilize techniques that are well known in the art. For some tissues, such as cardiac tissue, special methods may need to be employer. For example, it may be desirable to stabilize the cardiac tissue while the scaffolds are implanted. This can be done by any of a number of ways, including the induction of asystole of the heart or stabilizing a local region of the heart receiving the scaffold. It is also desirable for the self-fixating scaffold to be deliverable endocardially, transvascularly, or epicardially to the cardiac tissue.

Although the present invention has been described with reference to preferred embodiments, workers skilled in the art will recognize that changes may be made in form and detail without departing from the scope of the invention as defined in the appended claims.

## Claims

1. A self-fixating scaffold device for implantation into body tissue comprising:
a scaffold (32) for holding a therapeutic material; and **characterized by**
a skeleton (34) removably implanted within the scaffold;
wherein the scaffold changes form upon removal of the skeleton to anchor the scaffold in tissue.

2. The self-fixating scaffold of claim 1 wherein the scaffold is biodegradable.

3. The self-fixating scaffold of claim 2 wherein the scaffold is fabricated from at least one of: PLA, PGA, PLGA, PEG, PCL, PLLA, polyurethane-PCL, polyurethane-PEO, PCLA, alginate, collagen, starch/cellulose, cellulose acetate, fibrin, platelet gel, hydrogel, gelatin, chitin, pectin, and hyaluronic acid.

4. The self-fixating scaffold of claim 1 wherein the therapeutic material is biological material.

5. The self-fixating scaffold of claim 1 wherein the therapeutic material is a pharmaceutical drug.

6. The self-fixating scaffold of claim 1 wherein the therapeutic material is impregnated within the scaffold prior to anchoring the scaffold in the tissue.

7. The self-fixating scaffold of claim 1 wherein the therapeutic material is deliverable to the scaffold after the scaffold is anchored in the tissue.

8. The self-fixating element of claim 1 wherein the scaffold has a lattice structure for accomplishing directed cell growth.

9. The self-fixating scaffold device of claim 1 wherein the skeleton is fabricated from a metallic material.

10. The self-fixating scaffold of claim 1 wherein the skeleton comprises a lead for remote control implantation.

## Patentansprüche

1. Selbstfixierende Gerüstvorrichtung zur Implantation in Körpergewebe mit:
einem Gerüst (32) zum Halten eines therapeutischen Materials; und **gekennzeichnet durch**
ein Skelett (34), das entfernbar in dem Gerüst implantiert ist;
wobei das Gerüst auf das Entfernen des Skeletts hin seine Form verändert, um das Gerüst in Gewebe zu verankern.

2. Selbstfixierendes Gerüst nach Anspruch 1, wobei das Gerüst bioabbaubar ist.

3. Selbstfixierendes Gerüst nach Anspruch 2, wobei das Gerüst wenigstens aus einem der Folgenden hergestellt ist: PLA, PGA, PLGA, PEG, PCL, PLLA, Polyurethan-PCL, Polyurethan-PEO, PCLA, Alginat, Kollagen, Stärke/Zellulose, Zelluloseacetat, Fibrin, Plättchengel, Hydrogel, Gelatine, Chitin, Pektin und Hyaluronsäure.

4. Selbstfixierendes Gerüst nach Anspruch 1, wobei das therapeutische Material biologisches Material ist.

5. Selbstfixierendes Gerüst nach Anspruch 1, wobei das therapeutische Material ein pharmazeutisches Medikament ist.

6. Selbstfixierendes Gerüst nach Anspruch 1, wobei das therapeutische Material in dem Gerüst vor dem Verankern des Gerüsts in dem Gewebe imprägniert wird.

7. Selbstfixierendes Gerüst nach Anspruch 1, wobei das therapeutische Material an das Gerüst bereitstellbar ist, nachdem das Gerüst in dem Gewebe verankert ist.

8. Selbstfixierendes Element nach Anspruch 1, wobei das Gerüst eine Gitterstruktur aufweist, um ein gerichtetes Zellwachstum zu erreichen.

9. Selbstfixierende Gerüstvorrichtung nach Anspruch 1, wobei das Skelett aus einem metallischen Material hergestellt ist.

10. Selbstfixierendes Gerüst nach Anspruch 1, wobei das Skelett eine Leitung zur ferngesteuerten Implantation aufweist.

## Revendications

1. Dispositif de matrice auto-fixante pour une implantation dans un tissu corporel, comportant :
une matrice (32) destinée à contenir une substance thérapeutique ; et **caractérisé par**
un squelette (34) implanté de manière amovible à l'intérieur de la matrice ;
dans lequel la matrice change de forme lors d'un retrait du squelette pour ancrer la matrice dans le tissu.

2. Matrice auto-fixante selon la revendication 1 dans laquelle la matrice est biodégradable.

3. Matrice auto-fixante selon la revendication 2 dans laquelle la matrice est fabriquée à partir d'au moins une substance parmi : PLA, PGA, PLGA, PEG, PCL, PLLA, polyuréthane-PCL, polyuréthane-PEO, PCLA, alginate, collagène, amidon/cellulose, acétate de cellulose, fibrine, gel de plaquettes, hydrogel, gélatine, chitine, pectine et acide hyaluronique.

4. Matrice auto-fixante selon la revendication 1 dans laquelle la substance thérapeutique est un matériau biologique.

5. Matrice auto-fixante selon la revendication 1 dans laquelle la substance thérapeutique est un médicament pharmaceutique.

6. Matrice auto-fixante selon la revendication 1 dans laquelle la substance thérapeutique est imprégnée à l'intérieur de la matrice avant d'ancrer la matrice dans le tissu.

7. Matrice auto-fixante selon la revendication 1 dans laquelle la substance thérapeutique peut être délivrée à la matrice après que la matrice ait été ancrée dans le tissu.

8. Elément auto-fixant selon la revendication 1 dans lequel la matrice a une structure réticulaire pour réaliser une croissance cellulaire dirigée.

9. Dispositif de matrice auto-fixante selon la revendication 1 dans lequel le squelette est fabriqué à partir d'un matériau métallique.

10. Matrice auto-fixante selon la revendication 1 dans laquelle le squelette comporte une conduite pour une implantation par télécommande.
